Europäisches Patentamt

(19) European Patent Office

Office européen des brevets

(11) Publication number: **0 099 752 B2**

# NEW EUROPEAN PATENT SPECIFICATION

(45) Date of publication of the new patent specification : **29.05.91 Bulletin 91/22**

(51) Int. Cl.⁵ : **C07C 231/06**

(21) Application number : **83304177.5**

(22) Date of filing : **19.07.83**

(54) Production of N-t-alkylamides from t-olefins or t-alcohols.

(30) Priority : **20.07.82 JP 127343/82
24.09.82 JP 167056/82
27.09.82 JP 169325/82**

(43) Date of publication of application :
**01.02.84 Bulletin 84/05**

(45) Publication of the grant of the patent :
**30.12.86 Bulletin 86/52**

(45) Mention of the opposition decision :
**29.05.91 Bulletin 91/22**

(84) Designated Contracting States :
**CH DE FR GB IT LI NL**

(56) References cited :
**DE-A- 1 965 004
DE-B- 1 231 685
GB-B- 1 198 680
US-A- 2 457 660
US-A- 2 719 176**

(56) References cited :
**US-A- 2 819 306
US-A- 2 819 307
US-A- 4 273 938
J. Am. Chem. Soc. 70 (1984) 4045
Org. Synthesis, Vol. 42 (1962), 16-18**

(73) Proprietor : **SUMITOMO CHEMICAL
COMPANY, LIMITED
Kitahama 4-chome 5-33
Chuo-ku Osaka 541 (JP)**

(72) Inventor : **Wake, Shigeo
No. 1241-66, Iioka
Saijo-shi Ehime (JP)**
Inventor : **Beppu, Masazo
No. 3-731, Ikku-cho 2-chome
Niihama-shi Ehime (JP)**
Inventor : **Mizuno, Tadashi
No. 20-1, Hoshigoe-cho
Niihama-shi Ehime (JP)**

(74) Representative : **Diamond, Bryan Clive et al
Gee & Co., Chancery House, Chancery Lane
London WC2A 1QU (GB)**

EP 0 099 752 B2

## Description

This invention relates to a process for producing N-t-alkylamides in high yield comprising reacting a combination of a tertiary olefin and water, and/or a tertiary alcohol with a nitrile in the presence of sulfuric acid.

It is known the N-*t*-alkylamides (hereafter simply referred to as TAA) are intermediates useful for producing mono-tertiary alkylamines which are raw materials of organic rubber chemicals or medicines.

For producing TAA, there have heretofore been known a process which comprises reacting tertiary olefins or tertiary alcohols and sulfuric acid with nitriles, generally in a liquid phase, under such conditions that a molar ratio of sulfuric acid to the tertiary olefins or tertiary alcohols is 1 : 1 to 2 : 1 and the molar ratio of the nitriles to the tertiary olefins or tertiary alcohols is 0.5 : 1 to 1 : 1, at temperatures of 20 to 60°C to thereby prepare a TAA-sulfuric acid addition product, then neutralizing the addition product with alkalis such as ammonia water or sodium hydroxide, etc. and isolating TAA (U.S. Patent 2,773,097 and *J. Amer. Chem. Soc.*, 70, 4045 (1948)), and a process which comprises reacting tertiary olefins or tertiary alcohols with nitriles at temperatures of 50 to 250°C in a liquid phase whilst the molar ratio of the nitriles to the tertiary olefins or tertiary alcohols is 0.6 : 1 to 1 : 1, in the presence of a catalyst of a 1 to 20 wt% aqueous acid solution to obtain TAA (U.S. Patent 2,457,660). The processes are, however, extremely unsatisfactory as industrial production since, for example, in the process in which sulfuric acid is employed in an amount of 1 to 2 molar times that of the tertiary olefins or tertiary alcohols, an equimolar amount of an alkali is required for isolating TAA and further the process involves a problem that waste water after neutralization must be treated ; further in the process in which a 1 to 20 wt% aqueous acid solution is employed, the yield of TAA is at best less than 20 mol%, as is mentioned in the working examples thereof.

In addition, a process using organic sulfonic acid type cation exchange resins (hereafter simply referred to as IER) as acid catalysts is described in U.S. Patent 4,273,938 as a process for producing TAA. However, the life of the catalysts is extremely short because IER is poisoned by mono-*t*-alkylamines or ammonia formed by decomposition of TAA produced or by ammonia formed by hydrolysis of HCN or due to polymerization of olefins within pores of IER. Further taking into account the cost of IER, the process is extremely unsatisfactory as a process for producing TAA on an industrial scale.

US Patent 2,819,307 discloses a process of making N-tertbutyl formamide by reacting the olefin isobutylene (1 mol) with hydrogen cyanide (1-1.3 moles) and sulfuric acid (1.5-2.5 moles) containing 13 to 18% of water ; the reagents are mixed and heated to 60°C and the mixture is neutralised. This reaction takes place in two stages as shown in the equations given ; and it is therefore necessary to use the molar excess of sulfuric acid.

US Patent 2,719,176 discloses a process of making N-tert-alkylamides by reacting, e.g., a tertiary alcohol with equimolar amount of a nitrile and 0.8-1.2 moles of sulfuric acid e.g. 80-90%, aqueous acid, but preferably as in the Example 5, 90-95% sulfuric acid diluted with 3-5 volumes of acetic acid. The reaction is again in two stages, and is carried out by mixing the reactants and flowing the mixture through a heat exchanger at a temperature of 45-95°C, and then neutralising. Although slightly less sulfuric acid can be used than in the process of USP 2819307, the use of acetic acid makes the neutralisation more difficult.

We have devised a simple method of making a tertiary alkylamide from either an olefin or alcohol, in a single reaction with the use of a small amount of sulfuric acid and on an industrial scale.

According to the invention we provide a process of producing an N-tert-alkylamide by reacting a tertiary olefin or tertiary alcohol with a nitrile and sulfuric acid in the liquid phase, at a temperature in the range of 50° to 150°C,
characterised in that the molar amounts used of the reactants, per one mole of the olefin or alcohol, are :

(a) a sulfuric acid 0.05-0.8 moles ;
(b) of nitrile 2 moles, and
(c) of water 0-8 to 1.5 moles per mole of olefin or 0.5 or less moles per mole of alcohol and that a single reaction step takes place and the process can be batchwise or continuous.

Specific examples of tertiary olefins which can be employed in the process of this invention include isobutylene, 2-phenylpropene, 2-benzylpropene, 2-methyl-1-butene, 2-methyl-2-butene, 2-methyl-1-pentene, 2-methyl-2-pentene, 2-methyl-1-hexene, 2-methyl-2-hexene, 2-methyl-1-heptene, 2-methyl-2-heptene, 2,4,4-trimethyl-1-pentene, 2,4,4-trimethyl-2-pentene.

Specific examples of tertiary alcohols which can be employed in the process of this invention include *t*-butyl alcohol, *t*-amyl alcohol, 2-phenyl-2-propanol, 1-phenyl-2-methyl-2-propanol, 2-methyl-2-pentanol, 2-methyl-2-hexanol, 2-methyl-2-heptanol, 2,4,4-trimethyl-2-pentanol, 2,4-dimethyl-2-pentanol, α,α-dimethylbenzyl alcohol. Specific examples of nitriles include hydrocyanic acid, acetonitrile, acrylonitrile.

The reaction temperature is 50 to 150°C, particularly 60 to 100°C. When the reaction temperature exceeds the above upper limit, the yield of TAA is lowered since not only TAA produced is liable to be decomposed to mono-*t*-alkylamines, further to tertiary olefins and/or tertiary alcohols but also sulfuric acid is poisoned by the thus formed mono-*t*-alkylamines or ammonia. Further when the reaction temperature is lower than the above lower limit, there is a tendency that the reaction rate becomes low and uneconomical.

The reaction pressure is not particularly limited and can be sufficient to be not less than the vapor pressure of the reaction starting materials and reaction products. If the pressure is more than the vapor pressure, a gas inert to the reaction, such as nitrogen, may also be employed.

It is necessary that sulfuric acid used in the process of this invention be in an amount of 0.05 to 0.8 molar, preferably 0.1 to 0.5 molar, that of the tertiary olefins and/or tertiary alcohols. If the amount of sulfuric acid used is smaller than the above lower limit, the conversion of the reaction becomes undesirably low. If the amount of sulfuric acid used is larger than the above upper limit, although no particular disadvantage is met in performing the reaction, since costs for neutralization treatment and waste water treatment increase, such is not preferred from an economical viewpoint.

The nitriles used in the process of this invention must be used in an amount of 2 to 10 molar, preferably 2 to 4 molar, that of the tertiary olefins and/or tertiary alcohols. When the amount of the nitriles used is smaller than the above lower limit, side reactions such as polymerization, etc. of the tertiary olefins occur much more so that selectivity of TAA becomes poor. Further when the amount of the nitriles used is too large, although no particular disadvantage appears in the reaction, it is not preferred from an economical viewpoint.

Excessive amounts of the nitriles used in the process of this invention can be repeatedly employed after recovery since most of them remain unreacted after completion of the reaction.

In case that the process of this invention is carried out using tertiary olefins as a starting material, an equimolar amount of water is theoretically required for the tertiary olefins ; in practice when tertiary olefins are employed as a starting material in the process of this invention, the amount of water is from 0.8 to 1.5 molar time that of the tertiary olefins ; if the amount of water used is less than 0.8 molar time, conversion of the reaction becomes low, and if the amount of water used exceeds 1.5 molar time, since concentration of sulfuric acid as a catalyst becomes too low so that not only progress of the reaction is delayed but also hydrolysis of the produced TAA occurs. On the other hand, in case of carrying out the process of this invention using tertiary alcohols as a starting material, it is not specifically required to add water. However, there is no inconvenience even though water in an amount of not greater than 0.5 molar time that of the tertiary alcohols is present in the system. In case that the amount of water added exceeds 0.5 molar time, since progress of the reaction is delayed and hydrolysis of TAA occurs, such is not preferred. The tertiary olefins and water are equilibrated with the tertiary alcohols in an acidic condition in the present invention and therefore, it is equivalent to employing the tertiary alcohols to use the tertiary olefins and water as the starting materials.

TAA obtained in accordance with the process of this invention can be taken out in a conventional manner such as phase separation, extraction or the like, after neutralizing sulfuric acid as a catalyst after completion of the reaction. Further, in case that mono-*t*-alkylamines are obtained by further hydrolyzing TAA, it is possible to isolate TAA once and then hydrolyze TAA with an alkali such as sodium hydroxide in a conventional manner; alternatively, it is also possible to perform hydrolysis by adding an alkali to the reaction mixture obtained by the process of this invention from which TAA has not been isolated. It is preferred that an excess of the nitriles remaining in the reaction system obtained by the process of this invention be removed in advance in a conventional manner such as degasification, or distillation after completion of the reaction before hydrolysis of TAA is conducted.

The process of this invention can be either batchwise or continuous.

As described above, by performing the reaction in accordance with this invention, N-*t*-alkylamides can be obtained in high yield and the amount of alkali required for neutralization and treatment of waste water can be greatly minimized and therefore, the process of this invention is extremely useful from an industrial viewpoint.

Hereafter this invention will be described in more detail with reference to the examples but is not deemed to be limited thereto.

In the examples, all percentages are molar unless otherwise indicated.

Example 1

In a 1 liter glass-made autoclave were charged 60 g (0.6 mol) of 98 wt% sulfuric acid, 54 g (3 mols) of water, 243 g (9 mols) of hydrocyanic acid and 168 g (3 mols) of isobutylene. While stirring, the mixture was reacted at 80°C for 2 hours. After distilling off the hydrocyanic acid remained in the reaction solution, the reaction solution was neutralized with sodium hydroxide. After the neutralization, the organic phase was subjected to gas chromatography analysis, N-*t*-Butylforamide (TBF) was obtained in an amount of 288 g (2.85 mols) and the yield was 95% based on isobutylene.

Example 2

The same procedure was repeated as in Example 1 except that 222 g (3 mols) of *t*-butyl alcohol was employed in place of 54 g (3 mols) of water and 168 g (3 mols) of isobutylene. TBF was obtained in an amount of 291 g (2.88 mols) and the yield was 96% based on *t*-butyl alcohol.

Example 3

The same procedure was repeated as in Example 1 except that 98 wt% sulfuric acid was employed in an amount of 20 g (0.2 mol) in place of 60 g (0.6 mol). TBF was obtained in an amount of 242 g (2.4 mols) and the yield was 80% based on isobutylene.

Comparative Example 1

The procedure was repeated as in Example 1 except that 98 wt% sulfuric acid was employed in an amount of 6 g (0.06 mol) in place of 60 g (0.6 mol). TBF was obtained in an amount of 118 g (1.17 mols) and the yield was 39% based on isobutylene.

Example 4

The same procedure was repeated as in Example 1 except that 98 wt% sulfuric acid was employed in an amount of 210 g (2.1 mols) in place of 60 g (0.6 mol). TBF was obtained in an amount of 287 g (2.84 mols) and the yield was 95% based on isobutylene.

Comparative Example 1A

The same procedure was repeated as in Example 1 except that hydrocyanic acid was employed in an amount of 122 g (4.5 mols) in place of 243 g (9 mols). TBF was obtained in an amount of 192 g (1.9 mols) and the yield was 63% based on isobutylene.

Comparative Example 2

The same procedure was repeated as in Example 1 except that hydrocyanic acid was employed in an amount of 81 g (3 mols) in place of 243 g (9 mols). TBF was obtained in an amount of 81 g (0.8 mol) and the yield was 27% based on isobutylene.

Example 6

The same procedure was repeated as in Example 1 except that hydrocyanic acid was employed in an amount of 315 g (15 mols) in place of 243 g (9 mols). TBF was obtained in an amount of 291 g (2.88 mols) and the yield was 96% based on isobutylene.

Example 7

The same procedure was repeated as in Example 1 except that hydrocyanic acid was employed in an amount of 504 g (24 mols) in place of 243 g (9 mols). TBF was obtained in an amount of 293 g (2.9 mols) and the yield was 97% based on isobutylene.

Example 8

The same procedure was repeated as in Example 1 except that water was employed in an amount of 72 g (4 mols) in place of 54 g (3 mols). TBF was obtained in an amount of 212 g (2.1 mols) and the yield was 70% based on isobutylene.

Comparative Example 3

The same procedure was repeated as in Example 1 except that water was employed in an amount of 108 g (6 mols) in place of 54 g (3 mols). TBF was obtained in an amount of 91 g (0.9 mol) and the yield was 30% based on isobutylene.

## Example 9

The same procedure was repeated as in Example except that the reaction temperature was changed from 80°C to 60°C. TBF was obtained in an amount of 242 g (2.4 mols) and the yield was 80% based on isobutylene.

## Example 10

The same procedure was repeated as in Example 1 except that the reaction temperature was changed from 80°C to 100°C. TBF was obtained in an amount of 245 g (2.43 mols) and the yield was 81 % based on isobutylene.

## Comparative Example 4

The same procedure was repeated as in Example 1 except that 105 g of IER (Amberlist-15, trademark) was employed in place of 60 g (0.6 mol) of 98 wt% sulfuric acid. TBF was obtained in an amount of 242.4 g (2.4 mols) and the yield was 80% based on isobutylene.

The reaction was further carried out under the same conditions, using IER separated by filtration. TBF was obtained in an amount of 106 g (1.05 mols) and the yield was 35% based on isobutylene.

## Example 11

In a 1.5 liter glass-made autoclave were charged 60 g (0.6 mol) of 98 wt% sulfuric acid, 369 g (9 mols) of acetonitrile and 222 g (3 mols) of t-butyl alcohol. While electromagnetically stirring, the mixture was reacted for 2 hours at 80°C. After neutralizing the reaction solution, the organic phase was subjected to gas chromatographic analysis. N-t-Butylacetamide was obtained in an amount of 328 g (2.85 mols) and the yield was 95% based on t-butyl alcohol.

## Example 12

The same procedure was repeated as in Example 11 except that 98 wt% sulfuric acid was employed in an amount of 20 g (0.2 mol) in place of 60 g (0.6 mol). N-t-Butylacetamide was obtained in an amount of 286 g (2.49 mols) and the yield was 83% based on t-butyl alcohol.

## Example 13

The same procedure was repeated as in Example 11 except that 98 wt% sulfuric acid was employed in an amount of 210 g (2.1 mols) in place of 60 g (0.6 mol). N-t-Butylacetamide was obtained in an amount of 327 g (2.84 mols) and the yield was 95% based on t-butyl alcohol.

## Comparative Example 5

The same procedure was repeated as in Example 11 except that 98 wt% sulfuric acid was employed in an amount of 6 g (0.06 mol) in place of 60 g (0.6 mol). N-t-Butylacetamide was obtained in an amount of 155 g (1.35 mols) and the yield was 45% based on t-butyl alcohol.

## Comparative Example 5A

The same procedure was repeated as in Example 11 except that acetonitrile was employed in an amount of 185 g (4.5 mols) in place of 369 g (9 mols). N-t-Butylacetamide was obtained in an amount of 220 g (1.91 mols) and the yield was 64% based on t-butyl alcohol.

## Comparative Example 6

The same procedure was repeated as in Example 11 except that acetonitrile was employed in an amount of 123 g (3 mols) in place of 369 g (9 mols). N-t-Butylacetamide was obtained in an amount of 86 g (0.75 mol) and the yield was 25% based on t-butyl alcohol.

Example 15

The same procedure was repeated as in Example 11 except that acetonitrile was employed in an amount of 615 g (15 mols) in place of 369 g (9 mols). N-t-Butylacetamide was obtained in an amount of 330 g (2.87 mols) and the yield was 96% based on t-butyl alcohol.

Example 16

The same procedure was repeated as in Example 11 except that acetonitrile was employed in an amount of 984 g (24 mols) in place of 369 g (9 mols). N-t-Butylacetamide was obtained in an amount of 335 g (2.91 mols) and the yield was 97% based on t-butyl alcohol.

Example 17

The same procedure was repeated as in Example 11 except that 18 g (1 mol) of water was additionally added. N-t-Butylacetamide was obtained in an amount of 242 g (2.1 mols) and the yield was 70% based on t-butyl alcohol.

Comparative Example 7

The same procedure was repeated as in Example 17 except that water was employed in an amount of 54 g (3 mols) in place of 18 g (1 mol). N-t-Butylacetamide was obtained in an amount of 106 g (0.92 mol) and the yield was 31% based on t-butyl alcohol.

Examples 18 to 20 and Comparative Example 8

The same procedure was repeated as in Example 11 except that the reaction temperature was changed from 80°C to 60°C, 100°C, 140°C and 160°C, respectively. The results are shown in the Table below.

TABLE

| | Reaction temperature (°C) | Yield of N-t-Butylacetamide (%) | Yield of N-t-Butylacetamide based on t-butyl alcohol (%) |
|---|---|---|---|
| Example 18 | 60 | 283 | 82 |
| 19 | 100 | 304 | 88 |
| 20 | 140 | 245 | 71 |
| Comparative Example 8 | 160 | 155 | 45 |

Example 21 The same procedure was repeated as in Example 11 except that 243 g (9 mols) of hydrocyanic acid and 264 g (3 mols) of t-amyl alcohol were employed in place of 369 g (9 mols) of acetonitrile and 222 g (3 mols) of t-butyl alcohol, respectively. N-t-Amylformamide was obtained in an amount of 317 g (2.76 mols) and the yield was 92% based on t-amyl alcohol.

Example 22

The same procedure was repeated as in Example 11 except that 477 g (9 mols) of acrylonitrile was employed in place of 369 g (9 mols) of acetonitrile. N-t-Butylacrylamide was obtained in an amount of 335 g (2.64 mols) and the yield was 88% based on t-butyl alcohol.

Example 23

The same procedure was repeated as in Example 21 except that 306 g (3 mols) of 2-methyl-2-pentanol was used in place of 264 g (3 mols) of t-amyl alcohol. N-(1,1-Dimethylbutyl)-formamide was obtained in an

6

amount of 352 g (2.73 mols) and the yield was 91% based on 2-methyl-2-pentanol.

Example 24

The same procedure was repeated as in Example 21 except that 348 g (3 mols) of 2,4-dimethyl-2-pentanol was used in place of 264 g (3 mols) of t-amyl alcohol. N-(1,1,3-Trimethylbutyl)-formamide was obtained in an amount of 389 g (2.72 mols) and the yield was 91% based on 2,4-dimethyl-2-pentanol.

Example 25

The same procedure was repeated as in Example 21 except that 408 g (3 mols) of α,α-dimethylbenzyl alcohol was used in place of 264 g (3 mols) of t-amyl alcohol. N-(1-Methyl-1-phenylethyl)-formamide was obtained in an amount of 335 g (2.06 mols) and the yield was 69% based on α,α-dimethylbenzyl alcohol.

Example 26

In a 1.5 liter glass-made autoclave were charged 60 g (0.6 mol) of 98 wt% sulfuric acid, 54 g (3 mols) of water, 369 g (9 mols) of acetonitrile and 168 g (3 mols) of isobutylene. While stirring by means of an electromagnetic type stirring device, the mixture was reacted at 80°C for 2 hours. After neutralizing the reaction solution, the organic phase was subjected to gas chromatographic analysis. N-t-Butylacetamide was obtained in an amount of 324 g (2.82 mols) and the yield was 94% based on isobutylene.

Example 27

The same procedure was repeated as in Example 26 except that 98 wt% sulfuric acid was used in an amount of 20 g (0.2 mol) in place of 60 g (0.6 mol). N-t-Butylacetamide was obtained in an amount of 283 g (2.46 mols) and the yield was 82% based on isobutylene.

Comparative Example 9

The same procedure was repeated as in Example 26 except that 98 wt% sulfuric acid was employed in an amount of 6 g (0.06 mol) in place of 60 g (0.6 mol). N-t-Butylacetamide was obtained in an amount of 138 g (1.2 mols) and the yield was 40% based on isobutylene.

Example 28

The same procedure was repeated as in Example 26 except that 98 wt% sulfuric acid was used in an amount of 210 g (2.1 mols) in place of 60 g (0.6 mol). N-t-Butylacetamide was obtained in an amount of 327 g (2.84 mols) and the yield was 95% based on isobutylene.

Comparative Example 9A

The same procedure was repeated as in Example 26 except that acetonitrile was used in an amount of 185 g (4.5 mols) in place of 369 g (9 mols). N-t-Butylacetamide was obtained in an amount of 224 g (1.95 mols) and the yield was 65% based on isobutylene.

Comparative Example 10

The same procedure was repeated as in Example 26 except that acetonitrile was used in an amount of 123 g (3 mols) in place of 369 g (9 mols). N-t-Butylacetamide was obtained in an amount of 86 g (0.75 mol) and the yield was 25% based on isobutylene.

Example 30

The same procedure was repeated as in Example 26 except that acetonitrile was used in an amount of 615 g (15 mols) in place of 369 g (9 mols). N-t-Butylacetamide was obtained in an amount of 331 g (2.88 mols) and the yield was 96% based on isobutylene.

EP 0 099 752 B2

Example 31

The same procedure was repeated as in Example 26 except that acetonitrile was used in an amount of 984 g (24 mols) in place of 369 g (9 mols). N-t-Butylacetamide was obtained in an amount of 334 g (2.9 mols) and the yield was 97% based on isobutylene.

Example 32

The same procedure was repeated as in Example 26 except that water was employed in an amount of 72 g (4 mols) in place of 54 g (3 mols). N-t-Butylacetamide was obtained in an amount of 242 g (2.1 mols) and the yield was 70% based on isobutylene.

Comparative Example 11

The same procedure was repeated as in Example 26 except that water was used in an amount of 108 g (6 mols) in place of 54 g (3 mols). N-t-Butylacetamide was obtained in an amount of 104 g (0.9 mol) and the yield was 30% based on isobutylene.

Example 33

The same procedure was repeated as in Example 26 except that the reaction temperature was changed from 80°C to 60°C. N-t-Butylacetamide was obtained in an amount of 282 g (2.45 mols) and the yield was 82% based on isobutylene.

Example 34

The same procedure was repeated as in Example 26 except that the reaction temperature was changed from 80°C to 100°C. N-t-Butylacetamide was obtained in an amount of 293 g (2.55 mols) and the yield was 85% based on isobutylene.

Example 35

The same procedure was repeated as in Example 26 except that the reaction temperature was changed from 80°C to 140°C. N-t-Butylacetamide was obtained in an amount of 242 g (2.1 mols) and the yield was 70% based on isobutylene.

Comparative Example 12

The same procedure was repeated as in Example 26 except that the reaction temperature was changed from 80°C to 160°C. N-t-Butylacetamide was obtained in an amount of 127 g (1.1 mols) and the yield was 37% based on isobutylene.

Example 36

The same procedure was repeated as in Example 26 except that 336 g (3 mols) of 2,4,4-trimethyl-1-pentene and 243 g (9 mols) of hydrocyanic acid were used in place of 168 g (3 mols) of isobutylene and 369 g (9 mols) of acetonitrile, respectively. N-t-Octylformamide was obtained in an amount of 424 g (2.7 mols) and the yield was 90% based on 2,4,4-trimethyl-1-pentene.

Example 37

The same procedure was repeated as in Example 26 except that 477 g (9 mols) of acrylonitrile was used in place of 369 g (9 mols) of acetonitrile. N-t-Butylacrylamide was obtained in an amount of 335 g (2.64 mols) and the yield was 88% based on isobutylene.

Example 38

The same procedure was repeated as in Example 36 except that 354 g (3 mols) of 2-phenylpropene was

8

used in place of 336 g (3 mols) of 2,4,4-trimethyl-1-pentene. N-1-Methyl-1-phenylethylformamide was obtained in an amount of 340 g (2.1 mols) and the yield was 70% based on 2-phenylpropene.

Example 39

The same procedure was repeated as in Example 36 except that 210 g (3 mols) of 2-methyl-2-butene was used in place of 336 g (3 mols) of 2,4,4-trimethyl-1-pentene. N-(1,1-Dimethylpropyl)-formamide was obtained in an amount of 316 g (2.75 mols) and the yield was 92% based on 2-methyl-2-butene.

Example 40

The same procedure was repeated as in Example 36 except that 294 g (3 mols) of 2-methyl-2-hexene was used in place of 336 g (3 mols) of 2,4,4-trimethyl-1-pentene. N-(1,1-Dimethylpentyl)-formamide was obtained in an amount of 386 g (2.70 mols) and the yield was 90% based on 2-methyl-2-hexene.

## Claims

1. A process of producing an N-tert-alkylamide by reacting a tertiary olefin or tertiary alcohol with a nitrile and sulfuric acid in the liquid phase, at a temperature in the range of 50° to 150°C, characterized in that the molar amounts used of the reactants, per one mole of the olefin or alcohol, are :
   (a) of sulfuric acid 0.05-0.8 moles ;
   (b) of nitrile, 2 to 10 moles, and
   (c) of water 0.8 to 1.5 moles per mole of olefin or 0.5 or less moles per mole of alcohol and that a single reaction step takes place and the process can be batchwise or continious.

## Ansprüche

1. Verfahren zur Herstellung von N-t-Alkylamiden durch Reaktion von t-Olefinen oder t-Alkoholen mit einem Nitril und Schwefelsäure in der flüssigen Phase und bei einer Temperatur im Gebiet von 50° bis 150°C, dadurch gekennzeichnet, dass die benützten molaren Menge von der Reaktanten, per Mol des Olefins oder des Alkohols :
   (a) 0,05-0,8 Mol von Schwefelsäure,
   (b) 2 bis 10 Mole von Nitril, und
   (c) 0,8 bis 1,5 Mole vom Wasser, per Mol vom Olefin, oder 0,5 oder weniger Mol per Mol vom Alkohol, sind, und dass eine mit einer einzigen Reaktion Stufe geschieht und das Prozess bei Sätze oder kontinueirlich sein kann.

## Revendications

1. Un procédé pour la production d'un N-tert-alkylamide par réaction d'une oléfine tertiaire ou d'un alcool tertiaire avec un nitrile et de l'acide sulfurique en phase liquide, à une température dans le domaine de 50° à 150°C, caractérisé en ce que les quantités molaires des réactifs utilisées par mole d'oléfine ou d'alcool sont :
   (a) 0,05-0,8 moles d'acide sulfurique ;
   (b) 2 à 10 moles de nitriles ; et
   (c) 0,8 à 1,5 moles d'eau par mole d'oléfine, ou bien 0,5 mole ou moins par mole d'alcool, et en ce qu'une étape réactionnelle unique a lieu, le procédé pouvant être réalisé par charges ou de manière continue.